# FASCICULE DE BREVET EUROPEEN

(11) **EP 0 926 115 B1**
(45) Date de publication et mention de la délivrance du brevet: **28.09.2005**
(21) Numéro de dépôt: 98204293.9
(22) Date de dépôt: 09.12.1998
(51) Int. Cl.: C07C 17/00, C07C 17/361, C07C 17/26, C07C 21/12

(54) **Procédé pour la production de perchloréthylène**
Verfahren zur Herstellung von Perchlorethylen
Process for the preparation of perchloroethylene

(30) Priorité: 15.12.1997 FR 9716050
(43) Date de publication de la demande: 30.06.1999
(73) Titulaire: SOLVAY (Société Anonyme), 1050 Bruxelles (BE)
(72) Inventeur: Monzain, Michel, 78510 Triel S/Seine (FR); Bouilhot, Eric, 39500 Tavaux (FR)
(74) Mandataire: Jacques, Philippe

(56) Documents cités:
- EP-A- 0 573 920
- FR-A- 2 120 181
- US-A- 5 426 256

## Description

La présente invention concerne un procédé pour la production de perchloréthylène par chloration thermique non catalytique d'hydrocarbures, d'hydrocarbures partiellement chlorés et/ou de leurs mélanges, combinée à la conversion en perchloréthylène de tétrachlorure de carbone (CCl₄) provenant de sources extérieures.

Lors de la fabrication de perchloréthylène par chloration thermique d'hydrocarbures ou d'hydrocarbures partiellement chlorés, des quantités importantes de CCl₄ sont généralement obtenues en tant que sous-produit. Suite au protocole de Montréal, l'utilisation industrielle du tétrachlorure de carbone est toutefois fortement limitée.

Il est connu que, selon les conditions opératoires, on peut orienter la réaction soit vers la production de tétrachlorure de carbone soit vers la production de perchloréthylène. La réaction d'équilibre suivante est connue entre le tétrachlorure de carbone et le perchloréthylène.

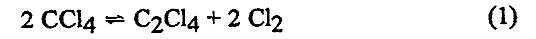

La production de perchloréthylène à partir de tétrachlorure de carbone via la réaction (1) s'accompagne toutefois généralement de la formation en quantités importantes de sous-produits à température d'ébullition élevée, tels que l'hexachlorobenzène.

La demande de brevet EP 0 573 920 propose un procédé de production de perchloréthylène à partir d'hydrocarbures et/ou d'hydrocarbures partiellement chlorés, de chlore et de tétrachlorure de carbone dans une installation réactionnelle maintenue à une température comprise entre 500 °C et 700 °C et sous une pression de 100-200 kPa afin d'obtenir un gaz dans lequel la quantité de CCl₄ est inférieure à la quantité de CCl₄ injectée et dans lequel la concentration de chlore est comprise entre 7 et 15 mol %. Le chlore et le CCl₄ contenus dans le gaz produit sont recyclés selon le procédé. Ce procédé ne permet toutefois pas de transformer des quantités importantes de CCl₄ extérieur, c'est-à-dire de CCl₄ non généré dans le procédé.

Le brevet US 5,426,256 décrit un procédé similaire dans lequel les réactifs sont introduits dans une installation réactionnelle via une zone de prémélange. Dans ce procédé, le tétrachlorure de carbone doit être introduit en partie à l'état liquide et en partie à l'état gazeux.

Le but de la présente invention est de proposer un procédé de production de perchloréthylène par chloration non catalytique permettant de transformer des quantités substantielles de tétrachlorure de carbone externe, sans générer des quantités importantes de sous-produits tels que l'hexachlorobenzène et qui évite les inconvénients des procédés antérieurs.

A cet effet, l'invention concerne un procédé pour la production de perchloréthylène par chloration thermique non catalytique d'hydrocarbures, d'hydrocarbures partiellement chlorés et/ou de leurs mélanges combinée à la conversion en perchloréthylène de tétrachlorure de carbone provenant de sources extérieures, le procédé comprenant les étapes suivantes:
a) production de perchloroéthylène par passage du tétrachlorure de carbone, des hydrocarbures en C₁-C₃, éventuellement partiellement chlorés, et de chlore dans une installation réactionnelle maintenue à une température de 500 à 700 °C et offrant un temps de séjour total de 5 à 25 s, la quantité de chlore introduite au réacteur étant réglée de manière à maintenir une concentration en chlore en sortie du réacteur de 3,5 à 6 % molaire,
b) séparation du perchloréthylène produit hors du mélange de produits réactionnels obtenu à l'étape a), et
c) recyclage du tétrachlorure de carbone et du chlore résiduels dans l'installation réactionnelle de l'étape a).

Ce procédé permet non seulement de convertir en C₂Cl₄ le CCl₄ généré lors de la chloration d'hydrocarbures mais également de convertir en perchloréthylène une quantité appréciable de CCl₄ externe, c.-à-d. en provenance d'autres procédés. On peut arriver ainsi en fonction des conditions opératoires et de la géométrie particulière de l'installation à convertir en perchloroétylène des quantités de CCl₄ qui représentent plus de 60 %, par exemple de 70 à 80 % de la quantité d'hydrocarbures consommée.

Le temps de résidence des réactifs dans l'installation réactionnelle est généralement au moins de 5 s. Habituellement, il ne dépasse pas 25 s. Typiquement, il est compris entre 5 s et 20 s. De préférence, il est compris entre 8 s et 16 s. De manière plus préférée, il est supérieur à 10 s.

On règle les rapports entre les différents réactifs introduits dans l'installation réactionnelle de telle sorte que, en sortie du réacteur, le rapport molaire HCl/Cl₂ est normalement au-dessus de 8. De manière préférée, il est supérieur à 10. En règle générale, ce rapport ne dépasse pas 20. Avantageusement, il ne dépasse pas 15. Il est typiquement compris entre 8 et 20, de préférence entre 10 et 15. La concentration en Cl₂ dans les produits de réaction est quant à elle généralement au moins de 3,5 % molaire. Habituellement, elle est au plus égale à 8 % molaire, et est avantageusement inférieure ou égale à 7 %. De manière préférée, elle est comprise entre 3,5 et 6,5 % molaire, de manière plus préférée entre 3,5 et 6 %, de manière tout particulièrement préférée entre 3,5 et 5 % molaire.

L'installation réactionnelle dans laquelle on réalise le procédé selon l'invention peut être constituée d'un ou de plusieurs réacteurs, comprenant eux-même une ou plusieurs zones de réaction. Par zone de réaction, on entend désigner une zone de l'installation dans laquelle la température est substantiellement homogène, c'est-à-dire où on n'observe pas d'écarts de température supérieurs à environ 10 °C. Dans un mode de réalisation préféré du procédé, le procédé se déroule dans plusieurs zone(s) de réaction.

Les conditions opératoires dans les différentes zones de réaction peuvent être optimisées en fonction des réactions qui s'y déroulent sans influencer directement les conditions opératoires dans les autres zones de réaction.

Selon un mode de réalisation préféré, le procédé se déroule dans deux zones de réaction distinctes, agencées avantageusement dans un réacteur unique. Avantageusement, la température de la deuxième zone de réaction (T₂) est inférieure à la température (T₁) de la première zone de réaction. La différence de température entre la première zone de réaction et la deuxième zone de réaction (T₁-T₂) est typiquement comprise entre 10 °C et 150 °C, de préférence entre 20 °C et 100 °C. Dans une forme particulière de ce mode de réalisation de l'invention, la deuxième zone de réaction n'est pas chauffée.

L'efficacité de la réaction, en particulier le taux de conversion du CCl₄ en C₂Cl₄, est influencée par la différence de température entre les deux zones de réaction. On a observé ainsi que plus la différence de température entre la première zone de réaction et la deuxième zone de réaction (T₁-T₂) est élevée, plus la conversion de tétrachlorure de carbone en perchloréthylène est importante.

Dans ce mode de réalisation, la température de la première zone de réaction se situe avantageusement entre 590 °C et 640 °C et la température de la deuxième zone de réaction se situe de préférence entre 550 °C et 600 °C.

Puisque les réactions qui se déroulent dans la deuxième zone de réaction sont largement endothermiques, il est opportun dans certains cas de prévoir un moyen de chauffer la deuxième zone de réaction de ladite installation. Dans ce mode de réalisation, le temps de résidence des réactifs dans chaque zone de réaction est normalement au moins de 1,5 s, et est typiquement compris entre 2 s et 15 s. De préférence, il est compris entre 2,5 s et 10 s, étant entendu que le temps de séjour total dans l'installation réactionnelle est toujours au moins de 5 s, de préférence au moins de 8 s.

Du tétrachlorure de carbone en provenance d'une source externe et/ou le tétrachlorure de carbone recyclé peut être injecté dans la première zone de réaction de ladite installation. Alternativement ou en combinaison avec l'injection dans la première zone de réaction, on peut également injecter du tétrachlorure de carbone en provenance d'une source externe et/ou le tétrachlorure de carbone recyclé dans la deuxième zone de réaction de ladite installation. En injectant du tétrachlorure de carbone dans la deuxième zone de réaction, on favorise plus encore la production de C₂Cl₄.

D'autres avantages et particularités de l'invention ressortiront de la description détaillée de modes de réalisation préférés décrits, à titre d'exemple, sur base des dessins en annexe, dans lesquels:

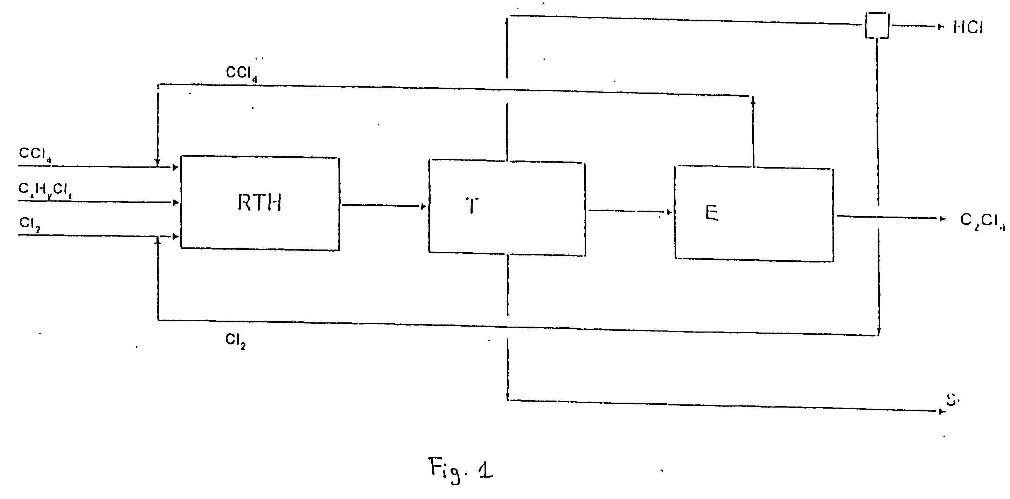

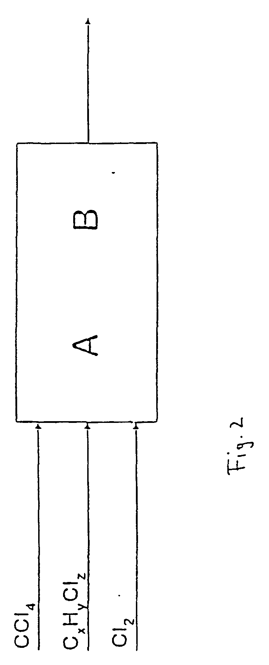

La Fig. 1 montre le schéma de principe du procédé pour la production de perchloréthylène et de chlorure d'hydrogène par chloration thermique non catalytique d'hydrocarbures, d'hydrocarbures partiellement chlorés et/ou de leurs mélanges en présence de tétrachlorure de carbone et par conversion de tétrachlorure de carbone en perchloréthylène. La Fig. 2 illustre le mode de réalisation préféré de l'invention, dans une installation comportant différentes zones de réaction dans un seul réacteur.

Le réacteur (RTH) est alimenté en tétrachlorure de carbone, en hydrocarbures, éventuellement en hydrocarbures partiellement chlorés (CₓH_{y}Cl_{z}) et en chlore (Cl₂).

Après la chloration thermique non-catalytique des hydrocarbures et la conversion du tétrachlorure de carbone en perchloréthylène et en chlore, le mélange gazeux est soumis à une trempe (T) et le chlorure d'hydrogène, le chlore et les sous-produits lourds (S) sont séparés du mélange. Les gaz résiduels sont ensuite soumis à une étape supplémentaire de séparation des impuretés et à une épuration (E) afin d'obtenir du perchloréthylène d'un degré de pureté voulu. Le tétrachlorure de carbone résiduel et le chlore résiduel sont recyclés au réacteur.

La Fig. 2 montre un mode de réalisation avantageux du présent procédé pour la production de perchloréthylène dans lequel deux zones de réaction se trouvent à l'intérieur d'un même réacteur. Dans la première zone de réaction A se déroule principalement la chloration thermique d'hydrocarbures en C₁-C₃, et/ou d'hydrocarbures en C₁-C₃ partiellement chlorés pour former du tétrachlorure de carbone et du perchloréthylène tandis que la conversion du tétrachlorure de carbone en perchloréthylène et en chlore a lieu principalement dans la deuxième zone de réaction B se trouvant en aval de la zone A.

### Exemple

Des essais de production de perchloréthylène par chloration thermique non catalytique de mélanges d'hydrocarbures et d'hydrocarbures partiellement chlorés en présence de tétrachlorure de carbone et par conversion de tétrachlorure de carbone en perchloréthylène dans une installation comportant au moins deux zones de réaction distinctes ont été réalisés.

En tant que matières premières, hormis le tétrachlorure de carbone et le chlore recyclé, les réactifs ont été mis en oeuvre dans les proportions suivantes:

| Réactif | débit (t/h) |
|---|---|
| CₓH_{y}Cl_{z} | 1,000 |
| CCl₄ extérieur | 0,643 |
| Cl₂ frais | 2,065 |

La composition moyenne du CₓH_{y}Cl_{z} était C: 30,0 %, H: 3,9 % et Cl 66,1 %.

L'installation a été exploité dans les conditions suivantes :

| | | |
|---|---|---|
| recyclages : | débit total | 3,186 t/h |
| | dont CCl₄ total | 2,800 t/h |
| | dont CCl₄ liquide | 0,326 t/h |
| températures | zone A | 615 °C |
| | zone B | 560 °C |
| Cl₂ en sortie du réacteur | | 3,6 % molaire |
| HCl/Cl₂ en sortie du réacteur | | 14,7 mol/mol |
| temps de séjour (total) | | 10,1 s |
| pression | | 1,95 bar abs |

Les produits suivants ont été retirés de l'installation :

| Produit | débit (t/h) |
|---|---|
| C₂Cl₄ | 2,061 |
| CCl₄ | 0 |
| HCI | 1,421 |
| Sous-produits | 0,226 |

## Revendications

1. Procédé pour la production de perchloréthylène au départ de tétrachlorure de carbone et d'hydrocarbures en C₁-C₃, éventuellement partiellement chlorés, comprenant les étapes de:
a) production de perchloroéthylène par passage du tétrachlorure de carbone, des hydrocarbures en C₁-C₃, éventuellement partiellement chlorés, et de chlore dans une installation réactionnelle maintenue à une température de 500 à 700°C et offrant un temps de séjour de 5 à 25 s, la quantité de chlore introduite au réacteur étant réglée de manière à maintenir une concentration en chlore en sortie du réacteur de 3,5 à 6 % molaire,
b) séparation du perchloréthylène produit hors du mélange de produits réactionnels obtenu à l'étape a), et
c) recyclage du tétrachlorure de carbone et du chlore résiduels dans l'installation réactionnelle de l'étape a).

2. Procédé selon la revendication 1, dans lequel le temps de séjour des réactifs dans l'installation est compris entre 8 et 16 s et la concentration en chlore en sortie du réacteur de 3,5 à 5 % molaire.

3. Procédé selon la revendication 1 ou 2, dans lequel l'installation réactionnelle comporte au moins deux zones de réaction distinctes.

4. Procédé selon la revendication 3, dans lequel la température de la deuxième zone de réaction (T₂) est inférieure à la température (T₁) de la première zone de réaction.

5. Procédé selon la revendication 3 ou 4, dans lequel la différence de température entre la première zone de réaction et la deuxième zone de réaction (T₁-T₂) est comprise entre 10 °C et 150 °C.

6. Procédé selon une quelconque des revendications 3 à 5, dans lequel la différence de température entre la première zone de réaction et la deuxième zone de réaction (T₁-T₂) est comprise entre 20 °C et 100 °C.

7. Procédé selon l'une quelconque des revendications 3 à 6, dans lequel la deuxième zone de réaction n'est pas chauffée.

8. Procédé selon l'une quelconque des revendications 3 à 7, dans lequel du tétrachlorure de carbone en provenance d'une source externe et/ou le tétrachlorure de carbone recyclé est injecté dans la première zone de réaction de ladite installation.

9. Procédé selon l'une quelconque des revendications 3 à 8, dans lequel du tétrachlorure de carbone en provenance d'une source externe et/ou le tétrachlorure de carbone recyclé est injecté dans la deuxième zone de réaction de ladite installation.

10. Procédé selon l'une quelconque des revendications 3 à 9, dans lequel, dans la première zone de réaction se déroule principalement la chloration thermique des hydrocarbures en C₁-C₃, éventuellement partiellement chlorés, pour former du tétrachlorure de carbone et du perchloréthylène, et dans la deuxième zone de réaction se déroule principalement la conversion du tétrachlorure de carbone en perchloréthylène et en chlore.

## Patentansprüche

1. Verfahren zur Herstellung von Perchlorethylen, ausgehend von Tetrachlorkohlenstoff und gegebenenfalls partiell chlorierten C₁-C₃-Kohlenwasserstoffen, das die folgenden Schritte umfasst :
a) Bildung von Perchlorethylen durch Einleiten von Tetrachlorkohlenstoff, von gegebenenfalls partiell chlorierten C₁-C₃-Kohlenwasserstoffen und von Chlor in eine Reaktionsanlage, die auf einer Temperatur von 500 bis 700°C gehalten wird und eine Verweilzeit von 5 bis 25 Sekunden ergibt, wobei die in den Reaktor eingeführte Chlormenge derart geregelt wird, dass am Reaktorausgang eine Chlorkonzentration von 3,5 bis 6 Mol-% aufrechterhalten wird,
b) Abtrennen des gebildeten Perchlorethylens aus dem in Schritt a) erhaltenen Gemisch von Reaktionsprodukten und
c) Recyclieren des restlichen Tetrachlorkohlenstoffs und des restlichen Chlors in die Reaktionsanlage von Schritt a).

2. Verfahren nach Anspruch 1, worin die Verweilzeit der Reaktanten in der Anlage zwischen 8 und 16 Sekunden beträgt und die Chlorkonzentration am Reaktorausgang von 3,5 bis 5 Mol-% beträgt.

3. Verfahren nach Anspruch 1 oder 2, worin die Reaktionsanlage wenigstens zwei verschiedene Reaktionszonen umfasst.

4. Verfahren nach Anspruch 3, worin die Temperatur der zweiten Reaktionszone (T₂) unter der Temperatur (T₁) der ersten Reaktionszone liegt.

5. Verfahren nach Anspruch 3 oder 4, worin der Temperaturunterschied zwischen der ersten Reaktionszone und der zweiten Reaktionszone (T₁-T₂) zwischen 10°C und 150°C beträgt.

6. Verfahren nach einem der Ansprüche 3 bis 5, worin der Temperaturunterschied zwischen der ersten Reaktionszone und der zweiten Reaktionszone (T₁-T₂) zwischen 20°C und 100°C beträgt.

7. Verfahren nach einem der Ansprüche 3 bis 6, worin die zweite Reaktionszone nicht beheizt wird.

8. Verfahren nach einem der Ansprüche 3 bis 7, worin Tetrachlorkohlenstoff aus einer externen Quelle und/oder recyclierter Tetrachlorkohlenstoff in die erste Reaktionszone der Anlage injiziert wird bzw. werden.

9. Verfahren nach einem der Ansprüche 3 bis 8, worin Tetrachlorkohlenstoff aus einer externen Quelle und/oder recyclierter Tetrachlorkohlenstoff in die zweite Reaktionszone der Anlage injiziert wird bzw. werden.

10. Verfahren nach einem der Ansprüche 3 bis 9, worin in der ersten Reaktionszone hauptsächlich die thermische Chlorierung der gegebenenfalls partiell chlorierten C₁-C₃-Kohlenwasserstoffe abläuft, um Tetrachlorkohlenstoff und Perchlorethylen auszubilden, und in der zweiten Reaktionszone hauptsächlich die Umwandlung des Tetrachlorkohlenstoffs zu Perchlorethylen und Chlor erfolgt.

## Claims

1. Process for the production of perchloroethylene from carbon tetrachloride and from C₁-C₃ hydrocarbons, which are optionally partially chlorinated, this process comprising the steps of :
a) production of perchloroethylene by passing carbon tetrachloride, C₁-C₃ hydrocarbons, which are optionally partially chlorinated, and chlorine into a reaction plant maintained at a temperature of from 500 to 700°C and involving a residence time of from 5 to 25 s, the amount of chlorine introduced into the reactor being controlled so as to maintain a chlorine concentration of from 3.5 to 6 mol% at the reactor outlet,
b) separation of the perchloroethylene produced from the mixture of reaction products obtained in step a), and
c) recycling of the residual carbon tetrachloride and chlorine into the reaction plant of step a).

2. Process according to Claim 1, in which the residence time of the reagents in the plant is between 8 and 16 s and the chlorine concentration at the reactor outlet is from 3.5 to 5 mol%.

3. Process according to Claim 1 or 2, in which the reaction plant includes at least two separate reaction zones.

4. Process according to Claim 3, in which the temperature in the second reaction zone (T₂) is lower than the temperature (T₁) in the first reaction zone.

5. Process according to Claim 3 or 4, in which the temperature difference between the first reaction zone and the second reaction zone (T₁-T₂) is between 10°C and 150°C.

6. Process according to any one of Claims 3 to 5, in which the temperature difference between the first reaction zone and the second reaction zone (T₁-T₂) is between 20°C and 100°C.

7. Process according to any one of Claims 3 to 6, in which the second reaction zone is not heated.

8. Process according to any one of Claims 3 to 7, in which carbon tetrachloride obtained from an external source and/or recycled carbon tetrachloride is injected into the first reaction zone of the said plant.

9. Process according to any one of Claims 3 to 8, in which carbon tetrachloride obtained from an external source and/or recycled carbon tetrachloride is injected into the second reaction zone of the said plant.

10. Process according to any one of Claims 3 to 9, in which the thermal chlorination of C₁-C₃ hydrocarbons, which are optionally partially chlorinated, to form carbon tetrachloride and perchloroethylene mainly takes place in the first reaction zone, and the conversion of the carbon tetrachloride into perchloroethylene and into chlorine mainly takes place in the second reaction zone.
